(19) Europäisches Patentamt
European Patent Office
Office européen des brevets

(11) Veröffentlichungsnummer: **0 045 910**
**B1**

(12) # EUROPÄISCHE PATENTSCHRIFT

(45) Veröffentlichungstag der Patentschrift: **10.10.84**

(21) Anmeldenummer: **81106017.7**

(22) Anmeldetag: **31.07.81**

(51) Int. Cl.³: **C 07 D 209/42**, C 07 D 405/12, C 07 D 209/12, C 07 D 209/14, A 61 K 31/40

(54) **Indolderivate und Verfahren zu ihrer Herstellung.**

(30) Priorität: **08.08.80 DE 3029980**

(43) Veröffentlichungstag der Anmeldung: **17.02.82 Patentblatt 82/7**

(45) Bekanntmachung des Hinweises auf die Patenterteilung: **10.10.84 Patentblatt 84/41**

(84) Benannte Vertragsstaaten: **AT BE CH DE FR GB IT LI LU NL SE**

(56) Entgegenhaltungen:
**BE - A - 628 441**
**DE - A - 2 830 211**
**DE - A - 2 905 877**
**DE - A - 2 925 448**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Michel, Helmut, Ziegelgasse 2a, D-6800 Mannheim 31 (DE)**
Erfinder: **Kampe, Wolfgang, Dr.rer.nat, Zedernstrasse 49, D-6805 Heddesheim (DE)**
Erfinder: **Ofenloch, Roland, Hibiscusweg 2, D-6143 Lorsch (DE)**

**EP 0 045 910 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Indolderivate der allgemeinen Formel I

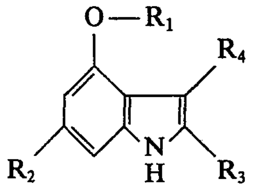

(I)

in welcher

$R_1$ Wasserstoff, Benzyl oder eine Gruppe

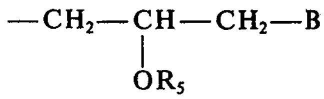

wobei B das Chlor- oder Bromatom, die Mesyloxy- oder Tosyloxygruppen bedeutet oder zusammen mit $R_5$ einen Valenzstrich und $R_5$ Wasserstoff oder einen Acetyl-, Formyl- oder Benzoylrest,
$R_2$ Wasserstoff oder eine $C_1-C_6$ Alkylgruppe,
$R_3$ Wasserstoff, eine Methylgruppe oder eine Gruppe $-CH_2-O-R_5$, wobei $R_5$ die oben genannte Bedeutung hat, und
$R_4$ Cyano

bedeuten.
Weiterer Gegenstand der Erfindung sind neue Indolderivate der allgemeinen Formel I'

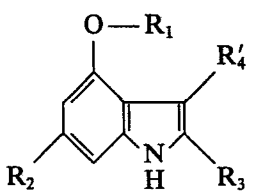

(I')

in welcher

$R_1$ Wasserstoff, Benzyl oder eine

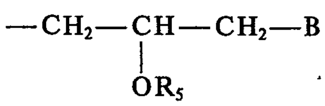

wobei B eine reaktive Gruppe darstellt oder zusammen mit $R_5$ einen Valenzstrich und $R_5$ Wasserstoff oder einen Acetyl-, Formyl- oder Benzylrest,
$R_2$ Wasserstoff oder eine $C_1-C_6$ Alkylgruppe,
$R_3$ Wasserstoff, eine Methylgruppe oder eine Gruppe $-CH_2-O-R_5$, wobei $R_5$ die oben genannte Bedeutung hat, und
$R_4'$ Aminocarbonyl, Oximinomethyl oder Formyl

bedeuten,
mit der Maßgabe, daß $R_1$ nicht die Bedeutung Benzyl hat, wenn $R_4'$ eine Formylgruppe darstellt.

Unter einer $C_1-C_6$ Alkylgruppe des Substituenten $R_2$ sind geradkettige oder verzweigte Gruppen vorzugsweise mit 1–4 Kohlenstoffatomen zu verstehen, wie z. B. Methyl, Ethyl, Propyl, Isopropyl, Butyl, Isobutyl, tert.-Butyl oder n-Hexyl. Insbesondere kommen jedoch die Methyl- oder tert.-Butylgruppen in Frage.

Die neuen Verbindungen der Formel I bzw. I' sind geeignete Zwischenprodukte zur Herstellung von Verbindungen mit wertvollen pharmazeutischen Eigenschaften, zum Beispiel zur Herstellung von

Verbindungen der allgemeinen Formel

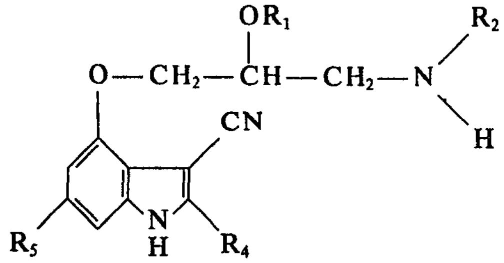

gemäß EP-A-0 045 911.

Hierbei werden die Verbindungen der Formel I direkt zu den Verbindungen der obigen Formel umgesetzt, während die Verbindungen der Formel I' zunächst nach üblichen Methoden in Verbindungen der Formel I überführt und dann weiterverarbeitet wurden. Diese Überführung kann beispielsweise durch die Umwandlung einer Formylgruppe in eine Oximinomethylgruppe durch Umsetzen mit Hydroxylamin sowie Dehydratisierung einer Aminocarbonyl- oder Oximinomethylgruppe zu einer Cyanogruppe geschehen.

Die Verbindungen der EP-A-0 045 911 zeigen eine Hemmung der $\beta_1$-Rezeptoren und sind somit ausgesprochen cardioselektiv. Sie eignen sich daher zur Behandlung oder Prophylaxe bei Herz- und Kreislauferkrankungen.

Die Herstellung der Verbindungen der allgemeinen Formel I geschieht in der Weise, daß man

a) eine Verbindung der Formel IV

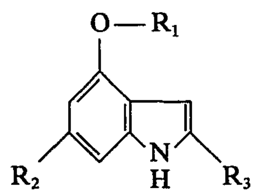

(IV)

in der $R_1$, $R_2$, $R_3$ die oben angegebenen Bedeutungen bedeuten,
mit
N-Carbonylsulfamoylchlorid, das auch als Chlorsulfonylisocyanat bezeichnet wird, in einem geeigneten Lösungsmittel nach an sich bekannten Verfahren (Chem. Ber. 100, 2719 (1967); Synthesis 1978, 374 und J. Chem. Soc., Perkin I, 1978, 1117)
oder

b) für den Fall, daß $R_1$ in der allgemeinen Formel I ein Wasserstoffatom oder eine Benzylgruppe bedeutet, eine Verbindung der Formel IV, in der $R_1$ Wasserstoff oder Benzyl darstellt und $R_2$ und $R_3$ die oben angegebenen Bedeutungen haben, mit
frisch hergestellten Triphenylphosphinisothiocyanat in einem geeigneten Lösungsmittel nach an sich bekannten Verfahren (Tetrahedron Lett. 1977, 4417 oder J. Chem. Soc. Perkin I, 1980, 1132)

umsetzt

und gegebenenfalls anschließend in einer erhaltenen Verbindung der allgemeinen Formel I einen der Reste $R_1$, $R_3$ oder $R_5$ nach üblichen Methoden in einen anderen, durch den Anspruch definierten Rest $R_1$, $R_3$ oder $R_5$ überführt sowie eine gegebenenfalls nach Verfahren a) erhaltene Aminocarbonylgruppe in die Cyanogruppe überführt.

Die erfindungsgemäßen Verfahren werden zweckmäßig in einem unter den Reaktionsbedingungen inerten Lösungsmittel, z. B. Wasser, Methanol, Ethanol, n-Butanol, Dioxan, Acetonitril, Nitromethan, Pyridin, Dimethylformamid oder Methylenchlorid, gegebenenfalls in Gegenwart eines säurebindenden Mittels durchgeführt.

Die Reaktionen gemäß Verfahren (a) werden unter Eiskühlung, bei Raumtemperatur oder unter Erwärmen, gegebenenfalls unter einer Schutzgasatmosphäre, ausgeführt.

Die Reaktionen nach (b) finden bei Temperaturen tiefer als $-20°C$ unter Schutzgasatmosphäre statt.

Die bei den erfindungsgemäßen Verfahren eingesetzten Ausgangsverbindungen sind in der Regel literaturbekannte Verbindungen. Neue Verbindungen werden im allgemeinen analog der für die Herstellung dieser bekannten Verbindungen beschriebenen erhalten.

Als gegebenenfalls nachträglich durchzuführende Umwandlung eines der Substituenten $R_1$, $R_3$

oder $R_5$ in Verbindungen der allgemeinen Formel I in einen anderen, durch den Anspruch 1 definierten Rest $R_1$, $R_3$ oder $R_5$ kommen beispielsweise
die Abspaltung einer Benzyloxygruppe zu einer Hydroxygruppe,
die Alkylierung einer Hydroxygruppe zu einer Epoxypropoxy-Verbindung in Frage.

In den folgenden Beispielen wird die Herstellung der erfindungsgemäßen Verbindungen näher beschrieben.

## Beispiel 1

### 4-(2,3-Epoxy-propoxy)-3-formylindol

9,9 g 4-Hydroxy-3-formylindol (Herst. Beispiel 3) werden in 200 ml Ethanol und 100 ml Epichlorhydrin gelöst, mit 6,4 g Natriummethylat versetzt und 6 h bei Raumtemperatur gerührt. Nach Abdampfen des Lösungsmittels wird mit Wasser und Ether gerührt, die Etherphase eingeengt und die verbleiobenden Kristalle abgesaugt. Man erhält 6,7 g (50% d. Th.) 4-(2,3-Epoxypropoxy)-3-formylindol vom Schmelzpunkt 108—110° C.

In analoger Weise erhält man:

| Bezeichnung | Ausbeute % | Schmelzp. Lösungsm. |
|---|---|---|
| a) 4-(2,3-Epoxy-propoxy)-3-aminocarbonylindol aus 4-Hydroxy-3-aminocarbonylindol (Beisp. 3a) | 80 | 70—75 (Sinterpunkt) (Essigester) |
| b) 4-(2,3-Epoxy-propoxy)-3-cyanindol aus 4-Hydroxy-3-cyanindol (Beisp. 3b) | 45 | 110—115 (Ether) |

## Beispiel 2

### 4-Benzyloxy-3-cyanindol

2,7 g 4-Benzyloxy-3-aminocarbonylindol (Beisp. 5) werden in 60 ml Pyridin (wasserfrei) mit 3,8 g p-Toluolsulfonylchlorid versetzt und nach Ende der Reaktion in 2 N Schwefelsäure gelöst. Die saure wäßrige Phase wird mit Ether extrahiert. Nach Eindampfen der Etherlösung verbleiben 1,5 g 4-Benzyloxy-3-cyanindol (60% d. Th.) vom Schmelzpunkt 139—141° C.

In analoger Weise erhält man:

| Bezeichnung | Ausbeute % | Schmelzp. Lösungsm. |
|---|---|---|
| a) 4-Benzyloxy-3-cyanindol aus 4-Benzyloxy-3-oximinomethylindol (Beisp. 4) | 40 | 139—140 (Ether) |
| b) 4-(2,3-Epoxy-propoxy)-3-cyanindol aus 4-(2,3-Epoxy-propoxy)-3-aminocarbonylindol (Beisp. 1b) | 20 | 116—120 (Ether) |

## Beispiel 3

### 4-Hydroxy-3-formylindol

17,2 g 4-Benzyloxy-3-formylindol (Lit: Can. J. Chem. 42 514 (1969)) werden in 700 ml Methanol gelöst, 5 ml Triethylamin zugegeben, mit 3 g 10%iger Palladium-Aktivkohle versetzt und bei Raumtemperatur

und 1 bar Wasserstoffdruck hydriert. Nach Entfernen des Katalysators wird eingeengt. Es verbleiben 11 g 4-Hydroxy-3-formylindol (98% d. Th.) vom Schmelzpunkt 196 – 200° C.

In analoger Weise werden erhalten:

| Bezeichnung | Ausbeute % | Schmelzp. Lösungsm. |
|---|---|---|
| a) 4-Hydroxy-3-aminocarbonylindol aus 4-Benzyloxy-3-aminocarbonylindol (Beisp. 5) | 85 | 304 – 305 (Methanol) |
| b) 4-Hydroxy-3-cyanindol aus 4-Benzyloxy-3-cyanindol (Beisp. 2, 2a, 6) | 56 | 205 – 207 (Methanol) |

Beispiel 4

4-Benzyloxy-3-oximinomethylindol

wird erhalten durch Umsetzung von 4-Benzyloxy-3-formylindol mit Hydroxyamin-Hydrochlorid und Natriumacetat in wäßriger Dimethylformamidlösung. Ausbeute 96% d. Th. Schmelzpunkt 206 – 210° C.

Beispiel 5

4-Benzyloxy-3-aminocarbonylindol

4,4 g 4-Benzyloxyindol (Lit:Helv. Chim. Acta 54 2411 (1971)) werden in 20 ml abs. Acetonitril gelöst, auf 0° C gekühlt und mit 1,8 ml N-Carbonylsulfamoylchlorid in 20 ml Acetonitril versetzt. Nach etwa 1 h Reaktionszeit gibt man eine Lösung von 3,4 g KOH in 34 ml Wasser zu und rührt 1 h bei Raumtemperatur nach. Nach Abdampfen des Lösungsmittels nimmt man in 200 ml 2 N Chlorwasserstoffsäure auf und extrahiert mit Methylenchlorid. Aus den Methylenchloridextrakten werden 4,8 g 4-Benzyloxy-3-aminocarbonylindol (45% d. Th.), aus Essigester umkristallisiert, vom Schmelzpunkt 176° C erhalten.

Beispiel 6

4-Benzyloxy-3-cyanindol

2,2 g 4-Benzyloxyindol in 10 ml Acetonitril werden bei 0° C mit 0,9 ml N-Carbonylsulfamoylchlorid in 10 ml Acetonitril versetzt.

Nach 1 h tropft man 1,4 ml Triethylamin in 5 ml Acetonitril zu, rührt 2 h bei Raumtemperatur nach und rührt in eiskalte Natriumhydrogencarbonatlösung ein. Nach Extraktion mit Ether verbleiben nach Eindampfen 1 g 4-Benzyloxy-3-cyanindol (40% d. Th.) vom Schmelzpunkt 139 – 141° C.

Beispiel 7

4-(2,3-Epoxy-propoxy)-3-cyanindol

7,7 g 4-(2,3-Epoxypropoxy)indol (Lit: Helv. Chim. Acta 54 2411 (1971)), gelöst in 150 ml Nitromethan, werden unter Kühlung bei 0° C – 5° C mit 3,9 ml N-Carbonylsulfamoylchlorid in 15 ml Nitromethan versetzt. Nach 30 Minuten Reaktionszeit gibt man 3,7 ml Dimethylformamid zu, stellt mit Triethylamin auf pH 9 und engt im Vaccum ein. Der Rückstand wird mit Essigester aufgenommen und über eine Kieselgelsäule mit Essigester gereinigt. Das Essigestereluat wird mit Wasser ausgeschüttelt, mit Natriumsulfat getrocknet und abgesaugt. Nach Entfernen des Lösungsmittels gibt man Ether zu und saugt ab. Es verbleiben 4,6 g 4-(2,3-Epoxy-propoxy)-3-cyanindol (54% d. Th.) vom Schmelzpunkt 116 – 120° C.

In analoger Weise erhält man:

| Bezeichnung | Ausbeute % | Schmelzp. Lösungsm. |
|---|---|---|
| a) 4-(2,3-Epoxy-propoxy)-2-acetoxymethyl-3-cyanindol aus 4-(2,3-Epoxy-propoxy)-2-acetoxymethylindol | 73 | 113–115 (Ether) |
| b) 4-(2,3-Epoxy-propoxy)-2-methyl-3-cyanindol aus 4-(2,3-Epoxy-propoxy)-2-methylindol (Lit: cit. Beisp. 7) | 57 | 137–138 (Ether) |
| c) 4-(2,3-Epoxy-propoxy)-3-cyan-6-methylindol aus 4-(2,3-Epoxy-propoxy)-6-methylindol (DE-OS 2 508 251) | 60 | Öl |
| d) 4-(2,3-Epoxy-propoxy)-3-cyan-6-t-butylindol aus 4-(2,3-Epoxy-propoxy)-6-t-butylindol | 70 | Öl |

Beispiel 8

4-Benzyloxy-3-cyanindol

Zu einer frisch bereiteten Lösung von 2,5 mmol Triphenyl-phosphinthiocyanat (Lit. Tetrahedron Letters 1977, S. 4417) tropft man unter Stickstoffatmosphäre bei −40° C eine Lösung von 1 mmol (2,2 g) 4-Benzyloxyindol in 20 ml absolutem Methylenchlorid zu und rührt noch 5 Stunden bei dieser Temperatur nach. Dann läßt man auf Raumtemperatur kommen und rührt über Nacht weiter. Nach Neutralisieren mit Triethylamin wird im Vakuum eingeengt und über eine Kieselgelsäule gereinigt. Nach Entfernen des Lösungsmittels verbleiben 1,5 g 4-Benzyloxy-3-cyanindol (60% d. Th.) vom Schmelzpunkt 138–140° C.

**Patentansprüche**

1. Indolderivate der allgemeinen Formel I

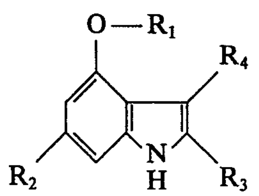

(I)

in welcher

$R_1$ Wasserstoff, Benzyl oder

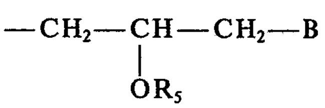

wobei B das Chlor- oder Bromatom, die Mesyloxy- oder Tosyloxygruppen bedeutet oder zusammen mit $R_5$ einen Valenzstrich und $R_5$ Wasserstoff oder einen Acetyl-, Formyl- oder Benzoylrest,

$R_2$ Wasserstoff oder eine $C_1-C_6$ Alkylgruppe,

$R_3$ Wasserstoff, eine Methylgruppe oder eine Gruppe $-CH_2-O-R_5$, wobei die oben genannte Bedeutung hat, und

$R_4$ Cyano

bedeuten.

2. Verbindungen der allgemeinen Formel I gemäß Anspruch 1, in denen $R_2$, $R_3$ und $R_4$ die angegebenen Bedeutungen haben und $R_1$ einen 2,3-Epoxypropylrest darstellt.

3. Verbindungen der allgemeinen Formel I gemäß einem der Ansprüche 1 oder 2, in denen $R_1$ und $R_4$ die angegebenen Bedeutungen haben und $R_2$ und $R_3$ Wasserstoff bedeuten.

4. Verbindungen der allgemeinen Formel I gemäß einem der Ansprüche 1 oder 3, in denen $R_2$, $R_3$ und $R_4$ die angegebenen Bedeutungen haben und $R_1$ einen Benzylrest darstellt.

5. Verfahren zur Herstellung von Verbindungen der allgemeinen Formel I gemäß Anspruch 1, dadurch gekennzeichnet, daß man in an sich bekannter Weise

a) eine Verbindung der allgemeinen Formel IV

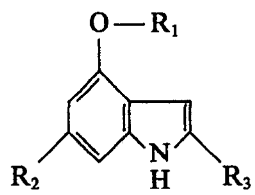

(IV)

in der $R_1$, $R_2$ und $R_3$ die angegebenen Bedeutungen haben,
mit
N-Carbonylsulfamoylchlorid in einem inerten Lösungsmittel
oder

b) für den Fall, daß $R_1$ in der allgemeinen Formel I ein Wasserstoffatom oder eine Benzylgruppe bedeutet,
eine Verbindung der Formel IV, in der $R_1$ Wasserstoff oder Benzyl darstellt und $R_2$ und $R_3$ die oben angegebenen Bedeutungen haben,
mit
frisch hergestelltem Triphenylphosphinisothiocyanat umsetzt und
gegebenenfalls anschließend in einer erhaltenen Verbindung der allgemeinen Formel I einen der Reste $R_1$, $R_3$ oder $R_5$ nach üblichen Methoden in einen anderen, durch den Anspruch definierten Rest $R_1$, $R_3$ oder $R_5$ überführt sowie eine gegebenenfalls nach Verfahren a) erhaltene Aminocarbonylgruppe in die Cyanogruppe überführt.

6. Verfahren nach Anspruch 5a), dadurch gekennzeichnet, daß man N-Carbonylsulfamoylchlorid und das Indolderivat der allgemeinen Formel IV in Nitromethan oder Acetonitril einsetzt und das Reaktionsgemisch nach Zugabe von Dimethylformamid mit einem tertiären Amin behandelt.

7. Indolderivate der allgemeinen Formel I'

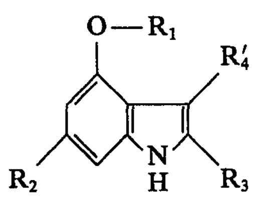

(I')

in welcher $R_1$, $R_2$, $R_3$ die im Anspruch 1 gegebenen Bedeutungen haben und $R_4'$ Aminocarbonyl, Oximinomethyl oder Formyl bedeuten,
mit der Maßgabe, daß $R_1$ nicht die Bedeutung Benzyl hat, wenn $R_4'$ eine Formylgruppe darstellt.

8. Verbindungen der allgemeinen Formel I' gemäß Anspruch 7, in denen $R_2$, $R_3$, $R_4'$ die angegebenen Bedeutungen haben und $R_1$ einen 2,3-Epoxypropylrest darstellt.

9. Verwendung von Verbindungen der allgemeinen Formel III

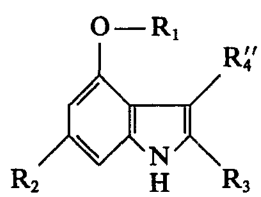

(III)

in welcher $R_1$, $R_2$, $R_3$ die im Anspruch 1 gegebenen Bedeutungen haben und $R_4''$ Amiinocarbonyl, Oxominomethyl, Formyl oder Cyano bedeuten,

zur Herstellung von Verbindungen der allgemeinen Formel II

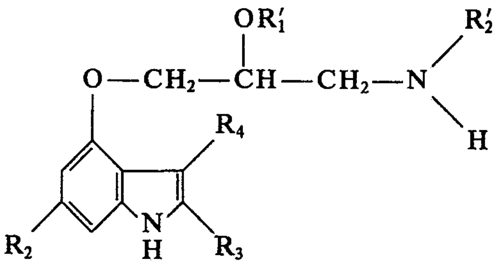

(II)

in welcher

$R_1$ Wasserstoff, eine Formyl-, Acetyl- oder eine Benzoylgruppe,
$R_2'$ eine $C_1-C_6$ Alkylgruppe oder
eine Gruppe

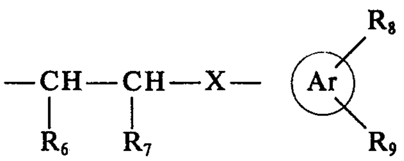

worin
X eine Bindung, eine Methylengruppe oder ein Sauerstoffatom,
Ar einen Phenylrest,
$R_6$ und $R_7$ gleich oder verschieden, jeweils Wasserstoff oder eine $C_1-C_6$ Alkylgruppe,
$R_8$ und $R_9$ gleich oder verschieden, jeweils Wasserstoff oder Halogen, eine Hydroxygruppe, eine $C_1-C_5$ Alkanoylgruppe, eine $C_2-C_4$ Alkenylgruppe, eine $C_2-C_4$ Alkinyloxygruppe, einen $C_1-C_6$ Alkylgruppe, eine $C_1-C_6$ Alkoxygruppe, eine $C_2-C_4$ Alkenyloxygruppe, eine $C_2-C_4$ Alkinyloxygruppe, eine $C_1-C_6$ Alkylthiogruppe, eine Aminocarbonylgruppe, sowie eine Acetamidogruppe bedeuten,
$R_3$ Wasserstoff, eine Methylgruppe oder eine Gruppe $-CH_2-O-R_5'$, wobei $R_5'$ Wasserstoff oder eine Acetyl-, Formyl- oder Benzoylgruppe bedeuten,
$R_4$ Cyano, und
$R_2$ Wasserstoff oder eine $C_1-C_6$ Alkylgruppe darstellt,
sowie deren pharmakologisch verträgliche Salze.

**Claims**

1. Indole derivatives of the general formula I

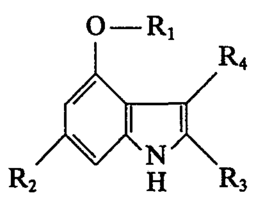

(I)

in which $R_1$ signifies hydrogen, benzyl or

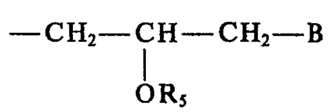

whereby B signifies a chlorine or bromine atom, the mesyloxy or tosyloxy group or, together with $R_5$, a valency bond and $R_5$ hydrogen or an acetyl, formyl or benzoyl radical, $R_2$ hydrogen or a $C_1-C_6$ alkyl group, $R_3$ hydrogen, a methyl group or a group $-CH_2-O-R_5$, whereby $R_5$ has the above-given meaning, and $R_4$ signifies cyano.

2. Compounds of general formula I according to claim 1, in which $R_2$, $R_3$ and $R_4$ have the given

meanings and $R_1$ represents a 2,3-epoxypropyl radical.

3. Compounds of general formula I according to one of claims 1 or 2, in which $R_1$ and $R_4$ have the given meanings and $R_2$ and $R_3$ signify hydrogen.

4. Compounds of general formula I according to one of claims 1 or 3, in which $R_2$, $R_3$ and $R_4$ have the given meanings and $R_1$ represents a benzyl radical.

5. Process for the preparation of compounds of general formula I according to claim 1, characterised in that, in per se known manner, one reacts

a) a compound of the general formula IV

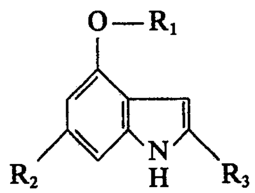

(IV)

in which $R_1$, $R_2$ and $R_3$ have the given meanings, with N-carbonylsulphamoyl chloride in an inert solvent; or

b) for the case in which $R_1$ in general formula I signifies a hydrogen atom or benzyl group, reacts a compound of formula IV, in which $R_1$ signifies hydrogen or benzyl and $R_2$ and $R_3$ have the above-given meanings, with freshly prepared triphenylphosphine thioisocyanate; and possibly subsequently, in a compound obtained of general formula I, converts one of the residues $R_1$, $R_3$ or $R_5$ according to usual methods into another residue $R_1$, $R_3$ or $R_5$ defined by the claim, as well as converts an aminocarbonyl group possibly obtained according to process a) into the cyano group.

6. Process according to claim 5a, characterised in that one places N-carbonylsulphamoyl chloride and the indole derivative of general formula IV into nitromethane or acetonitrile and treats the reaction mixture, after the addition of dimethylformamide, with a tertiary amine.

7. Indole derivatives of the general formula I'

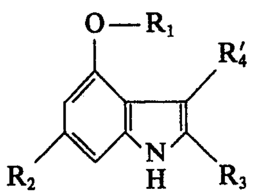

(I')

in which $R_1$, $R_2$, $R_3$ have the meanings given in claim 1 and $R_4'$ signifies aminocarbonyl, oximinomethyl or formyl, with the proviso that $R_1$ does not have the meaning benzyl when $R_4'$ represents a formyl group.

8. Compounds of the general formula I' according to claim 7, in which $R_2$, $R_3$, $R_4'$ have the given meanings and $R_1$ represents a 2,3-epoxypropyl radical.

9. Use of compounds of general formula III

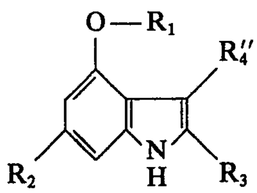

(III)

in which $R_1$, $R_2$, $R_3$ have the meanings given in claim 1 and $R_4''$ signifies aminocarbonyl, oximinomethyl, formyl or cyano, for the preparation of compounds of the general formula II

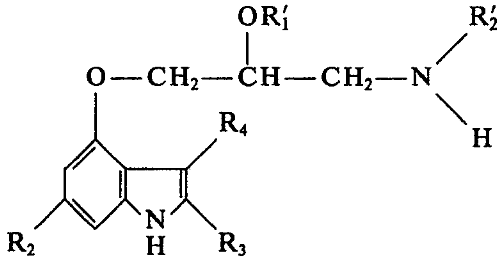

(II)

in which $R_1'$ represents hydrogen, a formyl, acetyl or a benzoyl group,
$R_2'$ represents a $C_1-C_6$ alkyl group of a group

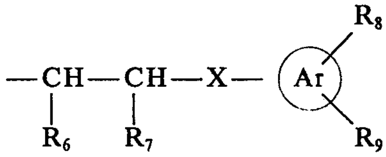

wherein
X signifies a bond, a methylene group or an oxygen atom, Ar a phenyl radical, $R_6$ and $R_7$ are the same or different, each representing hydrogen or a $C_1-C_6$ alkyl group, $R_8$ and $R_9$ are the same or different, each representing hydrogen or halogen, a hydroxyl group, a $C_1-C_5$ alkanoyl group, a $C_2-C_4$ alkenyl group, a $C_2-C_4$ alkynyl group, a $C_1-C_6$ alkyl group, a $C_1-C_6$ alkoxy group, a $C_2-C_4$ alkenyloxy group, a $C_2-C_4$ alkynyloxy group, a $C_1-C_6$ alkylthio group, an aminocarbonyl group, as well as an acetamido group, $R_3$ represents hydrogen, a methyl group or a group $-CH_2-OR_5'$, whereby $R_5'$ signifies hydrogen or an acetyl, formyl or benzoyl group,
$R_4$ represents cyano, and
$R_2$ represents hydrogen or a $C_1-C_6$ alkyl group, as well as their pharmacologically acceptable salts.

**Revendications**

1. Dérivés d'indol de formule générale I

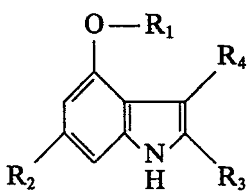

(I)

dans laquelle

$R_1$ est de l'hydrogène, du benzyle ou

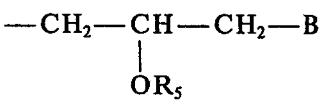

où B est l'atome de chlore ou de brome, ou le groupe mésyloxy ou tosyloxy, ou bien représente ensemble avec $R_5$ un trait de valence et $R_5$ est un reste acétyle, formyle ou benzoyle,
$R_2$ est de l'hydrogène ou un groupe alkyle $C_1-C_6$,
$R_3$ est de l'hydrogène, un groupe méthyle ou un groupe $-CH_2-O-R_5$, où $R_5$ a la signification donnée ci-dessus, et
$R_4$ est le groupe cyano.

2. Composés de formule générale I selon la revendication 1, dans lesquels $R_2$, $R_3$ et $R_4$ ont les significations indiquées et $R_1$ est un reste 2,3-époxypropyle.
3. Composés de formule générale I selon la revendication 1 ou 2 dans lesquels $R_1$ et $R_4$ ont les significations indiquées et $R_2$ et $R_3$ sont de l'hydrogène.
4. Composés de formule générale I selon la revendication 1 ou 3, dans lesquels $R_2$, $R_3$ et $R_4$ ont les significations indiquées et $R_1$ est un reste benzyle.
5. Procédé de préparation des composés de formule générale I définis dans la revendication 1, caractérisé en ce que de façon en soi connue on fait réagir:

a) un composé de formule générale IV

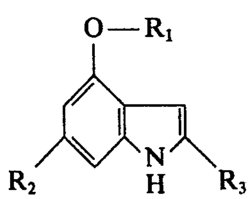

(IV)

dans laquelle $R_1$, $R_2$ et $R_3$ ont les significations indiquées,
avec
du chlorure de N-carbonylsulfamoyle dans un solvant inerte, ou

b) dans le cas où $R_1$ dans la formule générale I est un atome d'hydrogène ou un groupe benzyle, un composé de formule IV, dans lequel $R_1$ est de l'hydrogène ou du benzyle et $R_2$ et $R_3$ ont les significations ci-dessus indiquées,
avec
de l'isothiocyanate de triphénylphosphine fraîchement préparé, et, éventuellement on transforme ensuite dans un composé de formule générale I obtenu, un des restes $R_1$, $R_3$ ou $R_5$ selon un procédé habituel en un autre reste $R_1$, $R_3$ ou $R_5$ déffini dans la revendication, ainsi qu'un groupe aminocarbonyle éventuel obtenu selon le procédé a) en groupe cyano.

6. Procédé selon la revendication 5a) caractérisé en ce qu'on utilise le chlorure de N-carbonylsulfamoyle et le dérivé d'indol de formule générale IV dans du nitrométhane ou de l'acétonitrile et en ce qu'on traite le mélange réactionnel par une amine tertiaire après l'addition de diméthylformamide.

7. Dérivés d'indol de formule générale I':

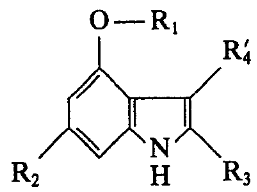

(I')

dans laquelle $R_1$, $R_2$ et $R_3$ ont les significations données dans la revendication 1 et
$R_4'$ est l'aminocarbonyle, l'oximinométhyle ou le formyle, sous la condition que $R_1$ n'est pas le benzyle quand $R_4'$ est un groupe formyle.

8. Composés de formule générale I' selon la revendication 7, dans lesquels $R_2$, $R_3$ et $R_4'$ ont les significations indiquées et $R_1$ est un reste 2,3-époxypropyle.

9. Utilisation de composés de formule générale III

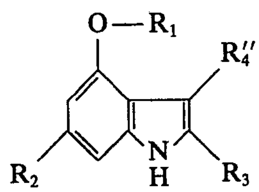

(III)

dans laquelle $R_1$, $R_2$ et $R_3$ ont les significations données dans la revendication 1 et,
$R_4''$ est l'aminocarbonyle, l'oximinométhyle, le formyle ou le cyano,
pour la préparation de composés de formule générale II

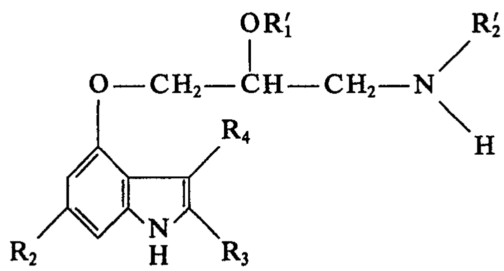

(II)

dans laquelle:

$R_1'$ est de l'hydrogène, un groupe formyle, acétyle ou benzoyle,
$R_2'$ est un groupe alkyle $C_1-C_6$ ou un groupe

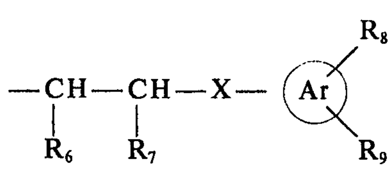

dans lequel
X est une liaison, un groupe méthylène ou un atome d'azote,
Ar est un reste phényle
$R_6$ et $R_7$ sont, identiques ou différents, chacun un atome d'hydrogène ou un groupe alkyle $C_1-C_6$,
$R_8$ et $R_9$ sont, identiques ou différents, chacun un atome d'hydrogène ou d'halogène, un groupe hydroxyle, un groupe alcanoyle $C_1-C_5$, un groupe alcényle $C_2-C_4$, un groupe alcinyle $C_2-C_4$, un groupe alkyle $C_1-C_6$, un groupe alcoxy $C_1-C_6$, un groupe alcényloxy $C_2-C_4$, un groupe alcinyloxy $C_2-C_4$, un groupe alkylthio $C_1-C_6$, un groupe aminocarbonyle ou aussi un groupe acétamido,
$R_3$ est de l'hydrogène, un groupe méthyle ou un groupe $-CH_2-O-R_5'$, où $R_5'$ est de l'hydrogène ou un groupe formyle ou benzoyle
$R_4$ est le groupe cyano et,
$R_2$ est de l'hydrogène ou un groupe alkyle $C_1-C_6$, ainsi que leurs sels pharmacologiquement tolérables.